# EUROPÄISCHE PATENTSCHRIFT

(11) **EP 3 010 425 B1**
(45) Veröffentlichungstag und Bekanntmachung des Hinweises auf die Patenterteilung: **08.12.2021**
(21) Anmeldenummer: 14732127.7
(22) Anmeldetag: 12.06.2014
(51) Int. Cl.: A61B 17/10, A61B 17/128, A61B 17/00, A61B 17/29, A61B 90/00

(54) **CHIRURGISCHER CLIP-APPLIKATOR**
SURGICAL CLIP APPLICATOR
INSTRUMENT DE POSE D'AGRAFES CHIRURGICALES

(30) Priorität: 17.06.2013 DE 102013106277
(43) Veröffentlichungstag der Anmeldung: 27.04.2016
(73) Patentinhaber: Aesculap AG, 78532 Tuttlingen (DE)
(72) Erfinder: SCHULZ, Peter, 79843 Löffingen (DE)
(74) Vertreter: Winter, Brandl - Partnerschaft mbB
(86) Internationale Anmeldenummer: PCT/EP2014/062193
(87) Internationale Veröffentlichungsnummer: WO 2014/202449

(56) Entgegenhaltungen:
- WO-A1-00/42922
- DE-U1-202011 000 755
- US-A- 4 611 595
- US-A1- 2008 083 813
- US-A1- 2011 024 145
- None

## Beschreibung

Die Offenbarung betrifft einen chirurgischen Clip-Applikator, ein Clip-Magazin für den Clip-Applikator sowie einen Handgriff zur lösbaren Aufnahme des Clip-Magazins.

### Hintergrund der Erfindung

Clip-Applikatoren sind chirurgische Instrumente zum Setzen (Applizieren) von Gewebeclips am Gewebe eines Patienten etwa zum Verschließen einer Naht oder eines Gefäßes. Dadurch entfällt ein aufwändiges Vernähen, was insbesondere bei von Außen schwer zugänglichen Operationsstellen vorteilhaft ist.

### Stand der Technik

Ein gattungsgemäßer Clip-Applikator ist beispielsweise aus der DE 20 2011 000 755 U1 bekannt. Diese Art von Clip-Applikator kann in der offenen und in der endoskopischen Chirurgie eingesetzt werden, um Gewebestrukturen eines zu operierenden Patienten durch ein Anlegen und anschließendes Schließen von vorzugsweise U-förmigen oder V-förmigen Clips schnell und zuverlässig zu verbinden. Insbesondere kann ein solcher Clip-Applikator dazu verwendet werden, Blutgefäße eines Patienten schnell und sicher abzuklemmen. Hierbei können verschiedene Cliptypen zum Einsatz kommen, die sich beispielsweise in ihrer Form, aber auch in ihrer Größe und Stärke, unterscheiden können.

Der in der DE 20 2011 000 755 U1 beschriebene Clip-Applikator deren Offenbarungsgehalt hiermit auch zum Gegenstand dieser Anmeldung gemacht wird, weist ein Handgriffteil auf, an dem ein Clip-Magazin mit einem integrierten Clipspeicher lösbar befestigt werden kann. Dabei ist das Handgriffteil dazu vorgesehen und entsprechend eingerichtet, um als wiederverwendbares Teil (Mehrweg-Teil) mehrfach benutzt zu werden. Dagegen ist das Clip-Magazin dafür vorgesehen und entsprechend eingerichtet, um als zu entsorgendes Teil (Einweg-Teil) nur solange verwendet zu werden, bis die im Clipspeicher des Clip-Magazins bevorrateten Clips aufgebraucht sind.

Obwohl dieser Clip-Applikator vergleichsweise kostengünstig bereitgestellt werden kann und auch ein vergleichsweise sicheres Anlegen und Schließen der Clips erlaubt, wäre es wünschenswert, die Handhabung des Clip-Applikators zu verbessern. Dieser Wunsch resultiert aus dem Umstand, dass das Handgriffteil dieses Clip-Applikators lediglich dafür vorgesehen ist, mit Clip-Magazinen derselben Bauart/der selben Clipart und Clipgröße kombiniert zu werden. In anderen Worten ausgedrückt, lässt sich mit ein und demselben Handgriffteil, trotz Austausch einzelner Clip-Magazine, nur genau ein bestimmter Cliptyp applizieren. Das heißt, dass im Operationssaal unter Umständen eine Vielzahl von verschieden Handgriffteilen sowie eine Vielzahl von verschiedenen Clip-Magazinen vorgehalten werden muss, von denen jede Kombination von Handgriffteil und Clip-Magazin für jeweils nur einen einzigen Cliptyp verwendet werden kann. Dies wirkt sich jedoch nachteilig auf die Platzverhältnisse und Übersichtlichkeit im Operationssaal aus.

Ein anderer Stand der Technik ist in der DE 44 29 084 C1 beschrieben. Der darin beschriebene Clip-Applikator ermöglicht ebenfalls ein vergleichsweise einfaches Auswechseln eines Clip-Magazins, das durch eine seitliche Durchbrechung in einen Rohrschaft des Clip-Applikators lösbar eingelegt werden kann. Dabei ist das Clip-Magazin als Einweg-Teil ausgebildet, während ein Handgriffteil und der daran befestigbare Rohrschaft als wiederverwendbare Teile ausgebildet sind. Weiter bietet dieser Clip-Applikator die Möglichkeit, unterschiedliche Rohrschäfte, die sich beispielsweise in ihrer Schaftlänge voneinander unterscheiden, an einem einzigen Handgriffteil zu befestigen. Obwohl dieser Clip-Applikator damit eine relativ flexible Handhabung erlaubt, ist es für die Applikation unterschiedlicher Cliptypen dennoch notwendig, sowohl den Rohrschaft, als auch das Clip-Magazin zu wechseln. Deshalb ist es weiterhin wünschenswert, die Handhabung des Clip-Applikators der eingangs beschriebenen Art zu verbessern.

Weiter ist aus der US 6 599 298 B1 ein Clip-Applikator bekannt, der ein Handgriffteil und ein daran befestigbares Clip-Magazin aufweist, die beide jeweils als Einweg-Teil ausgebildet sind. Dabei ist das Clip-Magazin in einen Schaft des Clip-Applikators integiert, wobei durch unterschiedliche Schaftlängen eine unterschiedliche Anzahl von Clips vorgehalten beziehungsweise gespeichert und gegebenenfalls appliziert werden kann. Allerdings bietet auch dieser Clip-Applikator keine Möglichkeit, unterschiedliche Cliptypen mit demselben Clip-Applikator zu applizieren, so dass weiterhin der Wunsch bestehen bleibt, die Handhabung des Clip-Applikators der eingangs beschriebenen Art zu verbessern.

Die WO 00/42922 A1 offenbart einen Clip-Applikator, zur Positionierung und Anbringung von chirurgischen Clips. Der Clip-Applikator besitzt einen Handgriff (pair of handles), mit Clipsen in einem wechselbaren Magazin.

Die US 2011/024145 A1 offenbart ein chirurgisches Gerät (Handgriff mit Betätigungselementen) für minimalinvasive Eingriffe und einer kraftbegrenzenden Kupplung. Eine Kupplung verhindert, dass am distalen Ende des chirurgischen Gerätes zu große Kräfte wirken können, die eventuell Schäden im Patienten hervorrufen.

Die US 2008/083813 A1 offenbart einen Handgriff für ein chirurgisches Instrument mit einer mechanischen Überlastbegrenzung.

Die US 4 611 595 A offenbart einen chirurgischen Clip-Applikator mit einem Handgriff, an den ein Magazin mit chirurgischen Clipsen angebracht werden kann. Das Clipmagazin ist wechselbar.

### Kurzbeschreibung der Erfindung

Ausgehend von der DE 20 2011 000 755 U1 liegt der Erfindung die Aufgabe zugrunde, einen vergleichsweise kostengünstig bereitstellbaren chirurgischen Clip-Applikator zu schaffen, bei dem unter Einsatz konstruktiv möglichst einfacher Mittel die Handhabung verbessert wird. Ein Ziel ist es hierbei, die Flexibilität des Clip-Applikators hinsichtlich der Einsatzmöglichkeit unterschiedlicher Cliptypen zu verbessern. Ein weiteres/anderes Ziel ist es, als Einweg-Teile konzipierte Bauteile des Clip-Applikators möglichst kostengünstig bereitstellen zu können.

Diese Aufgabe wird gelöst durch einen Universalhandgriff mit den Merkmalen des Anspruchs 1 sowie durch einen Clip-Applikator mit Universalhandgriff und Individual-Clipmagazin mit den Merkmalen des Anspruchs 5. Vorteilhafte Weiterbildungen der Erfindung sind in den Unteransprüchen angegeben.

Gemäß einem ersten Aspekt der vorliegenden Offenbarung wird folglich ein Universalhandgriff eines chirurgischen Clip-Applikators vorgeschlagen, an dem ein einen Crimpkopf aufweisendes, aus einer Anzahl von unterschiedlichen Clipmagazinen beliebig auswählbares Individual-Clipmagazin montierbar ist, derart, dass der Universalhandgriff und das daran befestigte Individual-Clipmagazin zum Vercrimpen jeweils einzelner Clips im Crimpkopf sowie zum Fördern jeweils einzelner Clips aus einem Clip-Aufnahmeabschnitt des Individual-Clipmagazins über einen individuellen Zustellweg hin zum Crimpkopf mechanisch zusammenwirken. Hierfür hat der Universalhandgriff ein integriertes Kraftübertragungsgetriebe bzw. Gestänge mit wenigstens einer manuell ergreifbaren Handhabe (Griffhebel, Scherengriffe, etc.) als Betätigungs-Eingangsteil und mit einer Kopplung als Betätigungs-Abgabeteil zum Individual-Clipmagazin. Es ist eine Adaptionsvorrichtung zwischen Betätigungs-Eingangsteil und Betätigungs-Abgabeteil vorgesehen, die zur Kompensation oder Anpassung von Clip-bedingter Unterschiede zwischen dem individuellen Zustellweg des aktuell befestigten Individual-Clipmagazins und dem maximalen Betätigungsweg des Betätigungs-Eingangsteils am Universalhandgriff angepasst ist.

Dadurch kann der Universalhandgriff mit einer Betätigungsmechanik ausgestattet sein, die einen bestimmten maximalen Betätigungsweg/-Betrag vorgesehen ist, welcher zur Applikation der für diesen maximalen Betätigungsweg/-Betrag geeignete Clips bestimmt ist. Sollen nunmehr auch Clips appliziert werden, die einen kleineren Betätigungsweg/-Betrag erfordern, findet durch die Adaptionsvorrichtung eine Kompensation oder Anpassung des maximalen Betätigungsweg/-Betrags mit Bezug auf den vom Individual-Clipmagazin definierten Betätigungsweg/-Betrag statt. Dadurch wird der Universalhandgriff auch für unterschiedliche Clips einsetzbar.

Vorteilhaft ist es, wenn die Adaptionsvorrichtung ein Überlastungsschutz für ein Unterbrechen der Kraftübertragung im Getriebe vorzugsweise in Form einer Rutschkupplung oder eines Ausklinkmechanismus ist. Insbesondere kann dann die Rutschkupplung auf einen Durchrutschwert oberhalb der für die Förderung einzelner Clips aus dem Clipmagazin über den individuellen Zustellweg hin zum Crimpkopf erforderlichen Kraft sowie unterhalb einer vorbestimmten, das Kraftübertragungsgetriebe überbelastenden Betätigungskraft eingestellt sein. Somit müssen an dem Kraftübertragungsgetriebe oder Gestänge keine Änderungen (Übersetzungswechsel, etc.) vorgenommen werden, sodass sich die Handhabung des Universalhandgriffs insgesamt vereinfacht.

Gemäß einem anderen Aspekt der vorliegenden Offenbarung wird ein chirurgischer Clip-Applikator vorgeschlagen, mit einem vorstehend beschriebenen Universalhandgriff und einem daran montierbaren Individual-Clipmagazin, das einen Crimpkopf, einen Clip-Aufnahmeabschnitt und eine Clip-Vorschubeinrichtung zum Fördern einzelner Clips aus dem Clip-Aufnahmeabschnitt über einen individuellen Zustellweg hin zum Crimpkopf in Abhängigkeit einer manuellen Betätigung des Universalhandgriffs aufweist. Dabei ist es vorgesehen, dass das aus einer Vielzahl von Clipmagazinen für unterschiedliche Cliptypen bedarfsweise auswählbare Individual-Clipmagazin eine integrierte und in Abhängigkeit des zu applizierenden Cliptyps ausgebildete Kodiermechanik aufweist, mit welcher wenigstens der an den jeweiligen Cliptyp angepasste Zustellweg festgelegt wird oder ist. Die Kodiermechanik hat zwei Begrenzungsanschläge, um die Bewegung der Clip-Vorschubeinrichtung in beide Längsrichtungen zu begrenzen. Die Clip-Vorschubeinrichtung ist dafür angepasst, um mit einem Rast-/Eingreifabschnitt am distalen Ende eines Betätigungs-Abgabeteils/Ausgangselements an einer Adaptionsvorrichtung des Universalhandgriffs zusammen zu wirken.

In anderen Worten, ist eine Kodiermechanik fest in die unterschiedlichen Clip-Magazine integriert, so dass mit einem universalen Handgriff unterschiedliche Clip-Magazine unterschiedlicher Baugröße/Baulänge und/oder mit unterschiedlichen Cliptypen verwendet werden können. Unter Zustellweg ist in diesem Zusammenhang der Transportweg für einzelne Clips aus einem Clipaufnahmeabschnitt/Clipspeicher des Clip-Magazins hin zum Crimpkopf zu verstehen.

Durch die Integration der Kodiermechanik zur Festlegung des an den jeweiligen Cliptyp angepassten Zustellwegs in das Clip-Magazin ist es möglich, an einem einzigen (universalen) Handgriff eine Vielzahl von unterschiedlichen Clip-Magazinen (lösbar) zu befestigen, diese bedarfsweise auszuwechseln und die darin jeweils bevorrateten Clips zu applizieren. Die im Wesentlichen schaftförmig ausgebildeten Clip-Magazine können unterschiedliche Schaftlängen aufweisen. Darüber hinaus können in den unterschiedlichen Clip-Magazinen auch jeweils unterschiedliche Cliptypen bevorratet sein, die sich beispielsweise in ihrer Form und/oder Größe und/oder Stärke voneinander unterscheiden. Das heißt, dass ein einziger (universaler) Handgriff mit einer Vielzahl, beispielsweise zwei, drei, vier, fünf oder mehr von unterschiedlichen Clip-Magazinen kombiniert werden kann, um unter Beibehaltung desselben Handgriffs unterschiedliche Cliptypen applizieren zu können.

Die Erfindung verbessert somit effektiv die Handhabung des Clip-Applikators und die Platzverhältnisse im Operationssaal, da mit einem einzigen Handgriff, lediglich durch eine entsprechende Auswahl und ein Auswechseln des Clip-Magazins unterschiedliche Cliptypen appliziert werden können bzw. unterschiedliche Schaftlängen des Clip-Applikators durch ein bloßes Auswechseln des Clip-Magazins realisiert werden können. Somit reduziert die Erfindung auch effektiv die Anzahl von unterschiedlichen Clip-Applikatoren, die im Operationssaal vorgehalten beziehungsweise bereitgestellt werden müssen. Auf diese Weise trägt die Erfindung zur Verbesserung der Übersichtlichkeit im Operationssaal bei.

Weiter bewirkt die in das jeweilige Clip-Magazin integrierte Kodiermechanik vorteilhafterweise, dass eine geringere Anzahl von wiederverwendbaren Handgriffen nach einer Operation sterilisiert werden müssen.

Vorzugsweise weist die Kodiermechanik eine Clip-Vorschubeinrichtung mit wenigstens einem in Längsrichtung der Clip-Vorschubeinrichtung wirkenden Begrenzungsanschlag zur wenigstens teilweisen Festlegung des Zustellwegs auf. In anderen Worten, ist die Clip-Vorschubeinrichtung ein erster Bestandteil der Kodiermechanik, der den Zustellweg der einzelnen Clips sicherstellt. Beispielsweise kann die Clip-Vorschubeinrichtung als eine Clip-Vorschubstange ausgebildet sein, die dazu eingerichtet beziehungsweise konfiguriert ist, dass bei jeder manuellen Betätigung des Handgriffs durch einen Benutzer und infolge einer dadurch bewirkten Längsbewegung immer nur genau ein einziger Clip aus der Vielzahl von im Clip-Magazin bevorrateten Clips aufgenommen und hin zum beziehungsweise hinein in das Anlegewerkzeug transportiert wird. Insbesondere, kann durch den wenigstens einen Begrenzungsanschlag an der Clip-Vorschubeinrichtung eine Rückwärts- und/oder Vorwärtsbewegung der Clip-Vorschubeinrichtung in Längsrichtung des Schafts des Handgriffs begrenzt und dadurch die Aufnahme eines einzigen Clips aus dem Clip-Magazins und dessen Transport hin zum Crimpkopf sichergestellt werden. In diesem Fall kommt die handgriffseitige Adaptionsvorrichtung bei Anschlagen des Begrenzungsanschlags zur Wirkung. D.h. im Fall einer Rutschkupplung oder eines entsprechenden Ausklinkmechanismus wird eine weitere Betätigung des Griffhebels zugelassen, ohne dass diese auf das Individual-Clipmagazin weiterhin übertragen wird.

Ein anderer gegebenenfalls unabhängiger oder zusätzlicher Aspekt der Offenbarung sieht vor, dass die Clip-Vorschubeinrichtung zwei in Längsrichtung/Vorschubrichtung derselben voneinander beabstandete Begrenzungsanschläge aufweist, deren Positionen und/oder Abstand in Abhängigkeit des zu applizierenden Cliptyps so angepasst sind, dass durch eine Rückwärts- und/oder Vorwärtsbewegung der Clip-Vorschubeinrichtung jeweils genau ein einzelner Clip aus dem Clip-Magazin aufnehmbar und exakt bis zum Crimpkopf transportierbar ist. Die Position der Begrenzungsanschläge ist insbesondere von der zu applizierenden Clipgröße und/oder -stärke und/oder -form, sowie gegebenenfalls von der Anzahl vorzuhaltender Clips im Clip-Magazin und/oder dessen Schaftlänge abhängig.

Es hat sich als vorteilhaft erwiesen, wenn der wenigstens eine Begrenzungsanschlag als ein Schnitt- und/oder Biegeteil in die Clip-Vorschubeinrichtung integriert ist, um auf diese Weise ein vergleichsweise günstig bereitstellbares Clip-Magazin zur Verfügung zu stellen. Beispielsweise kann die Vorschubeinrichtung eine Art Clip-Vorschubstange sein, die aus einem Blech oder dergleichen kostengünstig gefertigt werden kann. Bei dem Blech kann der wenigstens eine Begrenzungsanschlag dann auf kostengünstige Weise ausgestanzt und gegebenenfalls aufgebogen werden. Alternativ dazu ist es auch denkbar, die Clip-Vorschubeinrichtung als Kunststoffteil mit integriertem Begrenzungsanschlag auszubilden.

Ein anderer gegebenenfalls unabhängiger oder zusätzlicher Aspekt der Offenbarung sieht vor, dass die Kodiermechanik des Clip-Magazins auch einen an den zu applizierenden Cliptyp angepassten Crimpkopf (Maulteil) aufweist. In anderen Worten, kann der an den jeweiligen Cliptyp konstruktiv angepasste Crimpkopf ein Bestandteil der Kodiermechanik des Clip-Magazins sein, um das korrekte Schließen der Clips beim Applizieren derselben sicherzustellen. Das heißt, dass die Kodiermechanik alternativ oder zusätzlich zu dem an der Clip-Vorschubeinrichtung ausgebildeten Begrenzungsanschlag auch einen auf den zu applizierenden Cliptyp abgestimmten Crimpkopf aufweisen kann. Dadurch ist es möglich, unterschiedliche Clipgrößen, - stärken oder -formen und/oder Schaftlängen ohne eine Anpassung des (universalen) Handgriffteils auf zuverlässige und sichere Weise anzulegen und zu schließen. In diesem Fall ist die Magazin interne Mechanik für das Öffnen und Schließen des Crimpkopfs ebenfalls entsprechend angepasst, um über den Handgriff betätigt zu werden. Vorteilhaft ist es dabei, wenn die Magazin interne Crimpkopf-Betätigungsmechanik einen für alle unterschiedlichen Magazine einheitlichen Betätigungsweg/-betrag erfordert, sodass deren Betätigung durch den angekoppelten Universalhandgriff keine Adaptionsvorrichtung wie bei der individuellen Vorschubvorrichtung notwendig macht.

In einer besonders vorteilhaften Ausführungsform der Offenbarung weist der Crimpkopf eine das Schließen und Öffnen steuernde Kulisse auf, deren Position und/oder geometrische Form an den zu applizierenden Cliptyp und/oder an die Schaftlänge des Clip-Magazins angepasst ist. Beispielsweise kann die Kulisse so an dem Crimpkopf positioniert oder geometrisch ausgeformt sein, dass sich die Schließbewegung des Crimpkopfs bei einem vergleichsweise großen Clip von der Schließbewegung des Crimpkopfs bei einem vergleichsweise kleinen Clip unterscheidet. Die Crimpkopf-Betätigungsmechanik am Magazin kann dann vereinheitlicht werden.

Es hat sich als vorteilhaft erwiesen, wenn der Crimpkopf des Clip-Magazins angepasst ist, um mit dem wenigstens einen Begrenzungsanschlag der Clip-Vorschubeinrichtung in Kontakt zu kommen, um dadurch die Rückwärts- und/oder Vorwärtsbewegung der Clip-Vorschubeinrichtung zu begrenzen. In anderen Worten, können der Crimpkopf des Clip-Magazins und die Clip-Vorschubeinrichtung des Clip-Magazins dahingehend zusammenwirken, dass immer nur genau ein einziger Clip aus dem Clip-Magazin aufgenommen und hin zum Anlegewerkzeug transportiert wird.

Gemäß einem anderen gegebenenfalls unabhängigen oder zusätzlichen Aspekt der Offenbarung weist der Handgriff den mit der Kodiermechanik des Clip-Magazins zusammenwirkenden zuvor genannten Überlastungsschutz auf, der bei einer manuellen Betätigung des Handgriffs eine Beschädigung des Handgriffs und/oder des daran befestigten Clip-Magazins aufgrund zu hoher Betätigungskräfte verhindert. Das heißt, dass die flexible Handhabung des universal einsetzbaren Handgriffteils zusätzlich dadurch verbessert wird, dass ein (mechanischer) Überlastungsschutz fest in den Handgriff integriert ist.

In einer besonders kostengünstigen Ausführungsform der Offenbarung kann der Handgriff als ein wiederverwendbares Teil ausgebildet sein. Das heißt, dass der mit unterschiedlichen Clip-Magazinen universal verwendbare Handgriff, nach einer Sterilisation, auch in mehreren Operationen weiter verwendet werden kann.

Aus hygienischen Gründen kann jedes aus der Vielzahl von Clip-Magazinen als ein Einweg-Teil ausgebildet sein.

Gemäß einem anderen gegebenenfalls unabhängigen oder zusätzlichen Aspekt der Offenbarung können der Handgriff und/oder der Clip-Magazin eine an den zu applizierenden Cliptyp angepasste Farb-/Symbolkodierung zur Visualisierung des mit dem Handgriff verwendbaren Clip-Magazins aufweisen. Beispielsweise kann ein Gehäuse des Clip-Magazins in einer Farbe eingefärbt sein, die einem bestimmten Cliptyp zugeordnet ist. Für eine eindeutige Zuordnung des Clip-Magazins zu einem damit verwendbaren Handgriff, kann auf dem Handgriff eine entsprechend zuordbare Farbkodierung angebracht sein. Durch diese Farbkodierung der zwei zusammenfügbaren Bestandteile des Clip-Applikators wird eine besonders intuitive Verwendung des Clip-Applikators ermöglicht.

Zusammenfassend sieht die Offenbarung demnach vor, dass ein chirurgischer Clip-Applikator aus dem Handgriff und dem Clip-Magazin besteht. Das Clip-Magazin ist als ein vom Handgriff getrenntes beziehungsweise separiertes Bauteil gefertigt/vorgesehen und kann bedarfsweise an diesem lösbar befestigt/montiert werden, wobei der Handgriff und das daran befestigte Clip-Magazin zusammenwirken, um ein Werkzeug zur Applikation einzelner Clips und einen festgelegten Zustellweg für den Cliptransport einzelner Clips aus dem Clipspeicher hin zum Crimpkopf des Magazins auszubilden. Offenbarungsgemäß weist das aus einer Vielzahl von unterschiedlichen Clip-Magazinen für jeweils unterschiedliche Cliptypen bedarfsweise auswählbare Clip-Magazin eine integrierte und in Abhängigkeit des zu applizierenden Cliptyps ausgebildete Kodiermechanik auf, die wenigstens den an den jeweiligen Cliptyp angepassten Zustellweg festlegt.

Der Handgriff kann dabei einen integrierten Schaft aufweisen, in den das Clip-Magazin wenigstens teilweise eingelegt und gegebenenfalls auch eingeclipst werden kann. In jedem Clip-Magazin aus der Vielzahl von Clip-Magazinen kann eine bestimmte Anzahl von Clips angeordnet beziehungsweise bevorratet sein. Die in dem ClipSpeicher bevorratete Anzahl einzelner Clips kann etwa fünf bis vierzig Clips, vorzugsweise jedoch 20 bis 30 Clips, betragen. Die einzelnen Clip-Magazine aus der Vielzahl von Clip-Magazinen unterscheiden sich untereinander dadurch, dass sie aufgrund ihrer Baugröße (Baulänge) eine unterschiedliche Anzahl von Clips aufnehmen können. Weitere Unterscheidungsmerkmale der einzelnen Clip-Magazine untereinander sind auch die Baugröße/Breite, die Bauform (U-förmig oder V-förmig) und die Stärke beziehungsweise Dicke der darin bevorrateten einzelnen Clips, die in dem jeweiligen Clip-Magazin aufgenommen werden können. Diese unterschiedlichen Clips können zur Unterscheidung beispielweise als "klein (englisch: small)", "mittel (englisch: medium)", "mittelgroß (englisch: medium-large)" oder "groß (englisch:large)" bezeichnet sein. Die Clip-Magazine aus der Vielzahl von Clip-Magazinen können sich aber auch in der zu erzielenden Schaftlänge beziehungsweise gesamten Applikator-Länge unterscheiden, die beispielweise 240 mm, 290 mm oder 340 mm betragen kann.

Der universale Handgriff kann beispielsweise dazu konfiguriert sein, mit einem Clip-Magazin kombiniert zu werden, das 20 Clips der Größe "klein" bevorratet und im miteinander kombinierten, das heißt zusammengebauten Zustand zu einer Applikator-Länge von 240 mm führt. Derselbe Handgriff kann aber gleichzeitig auch dazu konfiguriert sein, mit einem Clip-Magazin kombiniert zu werden, der 20 Clips der Größe "mittel" bevorratet und im miteinander kombinierten Zustand zu einer Applikator-Länge von 240 mm führt.

Nach diesem Kombinationsschema sind wenigstens und/oder beispielweise die nachfolgend tabellarisch dargestellten Kombinationen von universalen Handgriffen mit jeweiligen Clip-Magazinen für den Zusammenbau zu einem Clip-Applikator denkbar:

| Clip-Größe | Applikator-Länge / Anzahl der Clips | | |
|---|---|---|---|
| | Handgriff-Modell 1 | Handgriff-Modell 2 | Handgriff-Modell 3 |
| | Griff 240 mm | Griff 290 mm | Griff 340 mm |
| klein (englisch: small) | 240 mm / 20 Clips | | |
| Mittel (englisch: Medium) | 240 mm / 20 Clips | 290 mm / 20 Clips | |
| | | 290 mm / 20 Clips | |
| mittelgroß (englisch: medium-Large) | | 290 mm / 20 Clips | |
| Groß (englisch:large) | | | 340 mm / 20 Clips |

### Figurenbeschreibung

Die Erfindung wird nachstehend anhand eines bevorzugten Ausführungsbeispiels unter Bezugnahme auf die begleitenden Figuren näher erläutert. Es zeigen:
Figur 1 eine perspektivische Draufsicht auf einen erfindungsgemäßen Clip-Applikator, der aus einem Handgriff oder Handgriffteil und einem daran befestigbaren Clip-Magazin besteht,
Figur 2 eine perspektivische Draufsicht auf den erfindungsgemäßen Clip-Applikator aus Figur 1 in einem zusammengebauten Zustand, in dem das Clip-Magazin an dem Handgriffteil lösbar befestigt ist,
Figur 3 eine perspektivische Unteransicht eines Clip-Magazins, das zu Illustrationszwecken teilweise demontiert ist,
Figur 4 eine perspektivische Unteransicht eines Teilabschnitts eines Clip-Magazins, das zu Illustrationszwecken teilweise demontiert ist, und
Figur 5 eine perspektivische Seitenansicht eines erfindungsgemäßen Clip-Applikators, der aus einem Handgriffteil und einem daran befestigten Clip-Magazin besteht und zu Illustrationszwecken teilsweise demontiert ist.

Gleiche oder ähnliche Komponenten sind durchgängig mit gleichen Bezugszeichen versehen.

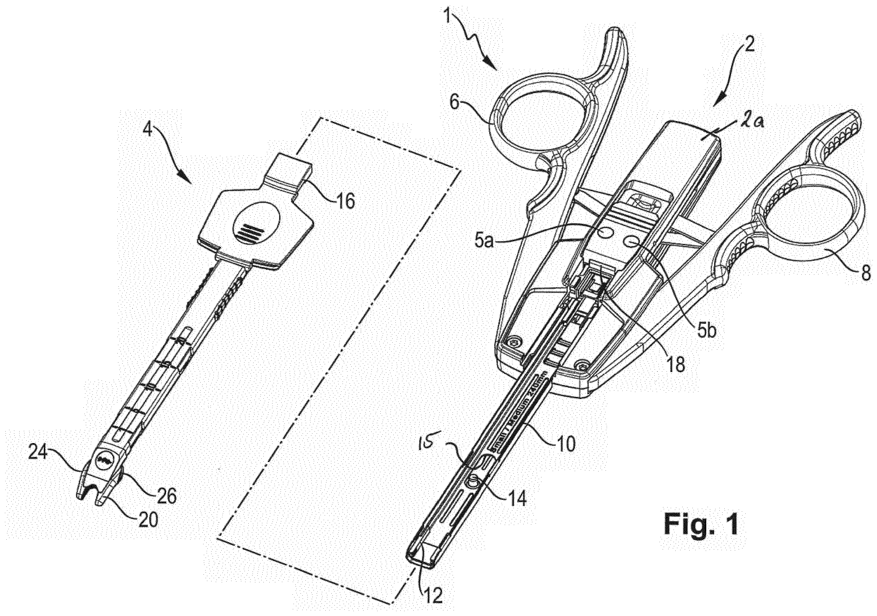

Figur 1 zeigt in einer perspektivischen Draufsicht allgemein einen erfindungsgemäßen Clip-Applikator (Clip-Applicator-Set) 1, der aus einem universalen (wiederverwendbaren) Handgriff 2 und einer Anzahl von jeweils einzel auswählbaren, individuellen (einmal verwendbaren) Clip-Magazinen besteht, von denen hier ein einzelnes Clip-Magazin 4 exemplarisch dargestellt ist. Ein solcher Clip-Applikator 1 kann beispielsweise in der offenen und/oder endoskopischen Chirurgie eingesetzt werden, um Gewebestrukturen eines Patienten schnell und zuverlässig miteinander zu verbinden beziehungsweise Blutgefäße eines Patienten schnell und zuverlässig abzuklemmen.

Der Hangriff 2 gemäß nachfolgender detaillierter Beschreibung und das im Wesentlichen schaftförmig ausgebildete Clip-Magazin 4 gemäß nachfolgender detaillierter Beschreibung sind generell jeweils dazu eingerichtet, dass das aus der Vielzahl von Clip-Magazinen bedarfsweise ausgewählte Clip-Magazin 4 an dem Handgriff 2 lösbar befestigt werden kann, um auf diese Weise gemeinsam den Clip-Applikator 1 auszubilden. Aus Illustrationszwecken sind der Handgriff 2 und das Clip-Magazin 4 hier getrennt voneinander dargestellt.

In jedem Clip-Magazin aus der Vielzahl von schaftförmigen Clip-Magazinen ist eine bestimmte Anzahl von Clips angeordnet beziehungsweise gespeichert / bevorratet. Üblicherweise sind in einem Clip-Magazin etwa fünf bis vierzig Clips, vorzugsweise jedoch 20 bis 30 Clips, angeordnet. Die einzelnen Clip-Magazine aus der Vielzahl von Clip-Magazinen unterscheiden sich untereinander dadurch, dass sie aufgrund ihrer Baugröße beziehungsweise Baulänge eine unterschiedliche Anzahl von Clips aufnehmen können. Weitere Unterscheidungsmerkmale der einzelnen Clip-Magazine untereinander sind auch die Baugröße/Breite, die Bauform (U-förmig oder V-förmig) und die Stärke beziehungsweise Dicke der einzelnen Clips, die in dem jeweiligen Clip-Magazin aufgenommen werden können. Die unterschiedlichen Clip-Magazine aus der Vielzahl von Clip-Magazinen können sich aber auch in ihrer Baulänge, insbesondere ihrer Schaftlänge unterscheiden.

Die bedarfsweise beziehungsweise bedarfsgerechte Auswahl eines bestimmten Clip-Magazins 4 aus der Vielzahl von Clip-Magazinen zur Befestigung an dem Handgriff 2 kann sich beispielsweise danach richten, welche Gewebestruktur im konkret vorliegenden Fall geklammert beziehungsweise geclipst werden soll, oder beispielsweise auch nach der Größe der abzuklemmenden Blutgefäße des Patienten. Um dem Anwender hierfür eindeutig anzuzeigen, mit welchem Handgriff 2 welches Clip-Magazin 4 verwendet werden kann/darf, weist der Handgriff 2 wenigstens an seiner dem Nutzer zugewandten Oberseite zwei Markierungen 5a und 5b auf, die den Handgriff 2 als ein für bestimmte Clip-Magazine 4 eingerichtetes, bzw. konfiguriertes Hangriffteil kennzeichnen. In diesem Ausführungsbeispiel sind die Markierungen 5a, 5b als kreisförmige, farbige Markierungen ausgebildet, die dem Nutzer/Anwender auf intuitive Weise anzeigen, um welche Ausführungsform des Handgriffteils es sich konkret handelt. In diesem Ausführungsbeispiel handelt es sich bei dem universalen Handgriff 2 um ein eingangs genanntes "Hangrifif-Modell 1" für die zu applizierende Clipgrößen "klein (englisch: small)" und/oder "mittel (englisch: medium)", das dementsprechend durch die zwei farbigen Markierungen 5a, 5b, beispielsweise gelb für "klein (englisch: small)" und blau für "mittel (englisch: medium)" angezeigt wird. Dementsprechend ist ein vorzugsweise aus einem Kunststoff gefertigtes Gehäuse des hierzu passenden Clip-Magazins 4 eingefärbt oder farblich markiert, nämlich in diesem Ausführungsbeispiel in gelber Farbe, um den im Clip-Magazin 4 gespeicherten beziehungsweise bevorrateten Cliptyp als "klein (englisch: small)" zu identifizieren.

### Konstruktion des Handgriffs

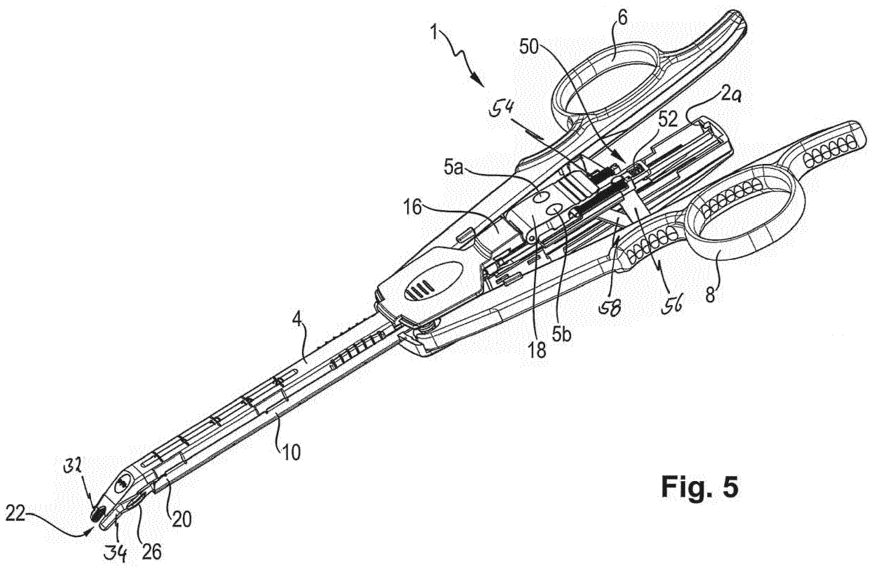

Gemäß der Figuren 1 und 5 weist der Handgriff 2 ein zentrales Gehäuse 2a zur Aufnahme eines Betätigungsgetriebes/Gestänges 54 auf, an dessen distalem Endabschnitt seitlich zwei Griffhebel/Scherengriffe 6 und 8 als Bedienungselemente zur manuellen Betätigung des Clip-Applikators 1 durch einen Anwender, beispielsweise einen Operateur oder Chirurgen, anscharniert sind, derart, dass sich die Griffhebel 6, 8 beidseits am zentralen Gehäuse 2a in Richtung proximal erstrecken. Die Griffhebel 6, 8 sind an ihren proximalen freien Enden jeweils an die Anatomie einer Greifhand des Anwenders konstruktiv angepasst/angenähert.

Des Weiteren weist der Handgriff 2 an seinem (aus Sicht eines Anwenders) distalen Endabschnitt einen mit dem Gehäuse 2a (stoff-) einstückig ausgebildeten oder an diesem befestigten, im Querschnitt U-förmigen Schaft 10 auf, in den eine Crimpauslöse- oder Schließvorrichtung 12 in Form eines längs verschiebbaren, U-förmig ausgebildeten Schiebers und ein Halteteil 14 in Form eines an dem Schaft 10 fixierten (angeschweißten) Bolzens integriert sind, der an einem Längsmittenabschnitt senkrecht vom Grund des U-förmigen Handgriff-Schafts 10 vorragt.

Die genaue Funktionsweise der Schließvorrichtung 12 und des Halteteils 14 werden nachstehend im Einzelnen erläutert. In Figur 1 ist jedoch zu erkennen, dass die als U-förmiger Schieber ausgebildete Schließvorrichtung 12 in deren Längsmittenabschnitt eine schlitzförmige Aussparung (Durchbruch) 15 aufweist, durch die der Bolzen 14 hindurchragt, wodurch eine Relativbewegung der Schließvorrichtung (U-förmiger Schieber) 12 gegenüber dem Bolzen 14 in Längsrichtung des Handgriff-Schafts 10 erlaubt wird.

Des Weiteren sind die Länge und die Breite des Schafts 10 des Handgriffs 2 so ausgebildet, dass das im Wesentlichen schaftförmig ausgebildete Clip-Magazin 4 wenigstens teilweise in den Schaft 10 derart eingelegt werden kann, dass der Handgriff 2 und das dazu passend ausgewählte Clip-Magazin 4 im zusammengebauten Zustand eine gesamte Clip-Applikator-Länge von 240 mm bis 340 mm, in diesem Ausführungsbeispiel von 240 mm, ergeben.

Gemäß der Fig. 1 und 5 ist am Gehäuse 2a des Handgriffs 2 ein von Außen betätigbarer Riegel 18 längsverschiebbar gelagert, der an seinem distalen Randabschnitt vorzugsweise eine Rastnase oder Leiste ausbildet. Der Riegel 18 ist dabei mittels einer in Fig. 1 nicht weiter dargestellten Feder 54 (siehe aber Fig. 5) in Richtung distal in eine Verriegelungsposition vorgespannt.

Das Gehäuse 2a hat an seinen beiden, den Griffhebeln 6, 8 jeweils zugewandten Seiten längsverlaufende Schlitze, in die jeweils zwei Pleuelstäbe (Zug-/Druckstäbe) 56, 58 eingeführt sind, welche die beiden Griffhebel 6, 8 mit dem Betätigungsgetriebe 54 des Handgriffs 2 bzw. mit der Schließvorrichtung 12 koppeln. Das Betätigungsgetriebe 54 besteht vorliegend aus einer relativ zur Schließvorrichtung 12 langsverschiebbar angeordneten, vorzugsweise riegelförmigen Mitnehmereinheit für eine nachfolgend noch beschriebene Clip-Vorschubeinrichtung eines eingesetzten Clip-Magazins 4.

Diese Mitnehmereinheit 54 hat vorzugsweise ein intern längsverschiebbar gelagertes Eingangselement, an dem jeweils ein Pleuelstab 56 zur Ankopplung beider Griffhebel 6, 8 an das Betätigungsgetriebe 54 angelenkt ist. Das Eingangselement ist über eine interne Rutschkupplung 52 mit einem relativ längsverschiebbar gelagerten Ausgangselement wirkverbunden, das an seinem distalen Ende einen Rast-/Eingreifabschnitt für die Clip-Vorschubeinrichtung des aktuell eingesetzten Clip-Magazins 4 hat. Die das Eingangselement mit den Griffhebeln 6, 8 koppelnden Pleuelstäbe 56 sind dabei ausgehend von den Griffhebeln 6, 8 in Richtung proximal orientiert, derart, dass bei einem scherengriffartigen Zusammendrücken der beiden Griffhebel 6, 8 das Eingangselement und damit über die Rutschkupplung 52 das Ausgangselement in Richtung proximal im Gehäuse 2a verschoben wird.

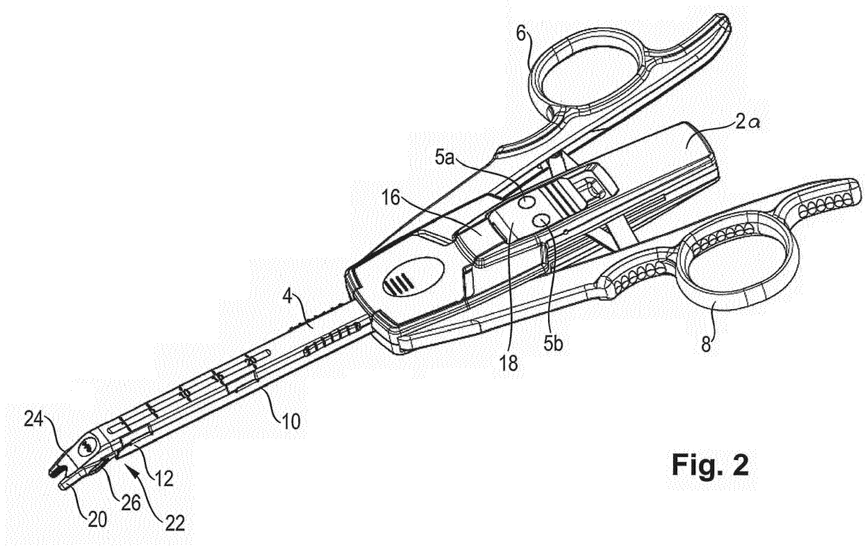

Dabei sei darauf hingewiesen, dass die gesamte Mitnehmereinheit 54 gemäß vorstehender Beschreibung über eine Feder am Gehäuse gehalten ist, sodass diese aus der proximalen Position (zusammengedrückte Griffhebel gemäß Fig. 2) in Richtung distale Position (gespreizte Griffhebel gemäß Fig. 1) elastisch bewegt wird. Ferner sei darauf hingewiesen, dass die Konstruktion der Mitnehmereinheit 54 auch anders ausgestaltet sein kann, solange eine Betätigungskraft, aufgebracht auf die Griffhebel 6, 8 über eine Adaptionsvorrichtung (Überlastungsschutz 50) entsprechend der beispielhaften Eingang-Ausgangselement-Rutschkupplung-Einheit auf den Rast-/Eingriffsabschnitt übertragen wird, um bei Überschreiten einer vorbestimmten Betätigungskraft den Kraftfluss zu unterbrechen und so die Griffhebel 6, 8 leer (kraftfrei) laufen zu lassen.

Beide Griffhebel 6, 8 sind über jeweils einen weiteren Pleuelstab 58 mit der Schließvorrichtung 12 in Form des im Hangriff-Schaft 10 gelagerten U-förmigen Schiebers gekoppelt. Diese beiden weiteren Pleuelstäbe 58 sind gegensätzlich zu den vorstehend genannten Pleuelstäben 56 orientiert, sodass bei einem scherengriffartigen Zusammendrücken der beiden Griffhebel 6, 8 der U-förmige Schieber 12 in Richtung distal verschoben wird.

### Konstruktion des Clip-Magazins

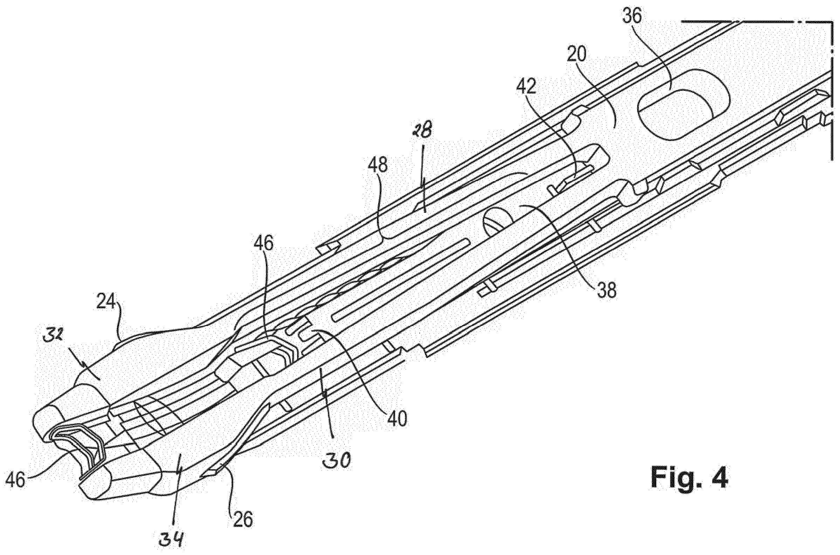

Jedes Clip-Magazin 4 gemäß der Fig. 1 und 4 hat einen Schaftabschnitt, der hinsichtlich seiner Abmessungen in den U-förmigen Schaft 10 des Handgriffs 2 eingesetzt werden kann und der auch gleichzeitig den Clipaufnahmeabschnitt (Clipspeicher) des Magazins 4 bildet. Zur lösbaren Befestigung/Arretierung an dem Handgriff 2 weist das im Wesentlichen schaftförmig ausgebildete Clip-Magazin 4 eine Montagevorrichtung 16 am proximalen Ende des Schaftabschnitts auf, die mit dem Riegel (Haltevorrichtung) 18 des Handgriffs 2 zusammenwirkt, um das (teilweise) in den Schaft 10 des Handgriffs 2 eingelegte Clip-Magazin 4 lösbar an dem Handgriff2 zu verriegeln/arretieren.

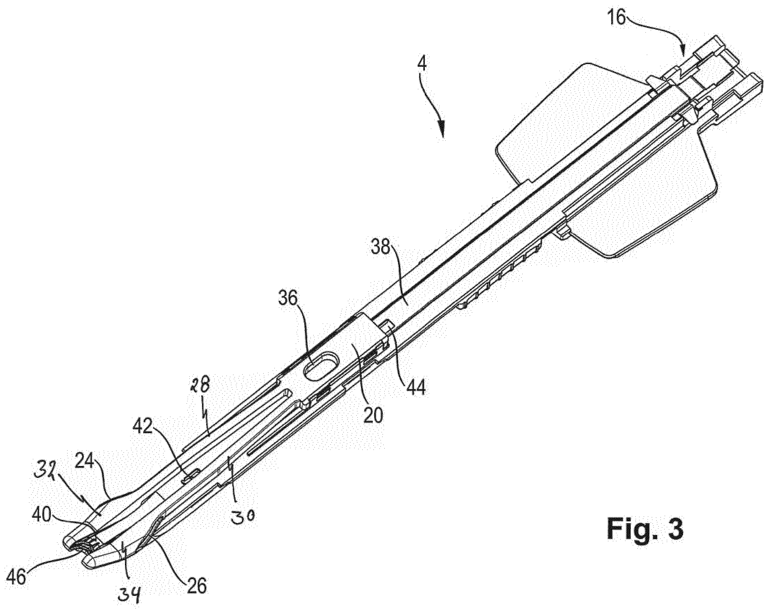

Am distalen Endschnitt des Schaftabschnitts des Clip-Magazins 4 ist gemäß der Fig. 3 und 4 ein Crimpwerkzeug 22 mit einem Crimpkopf (Maulteil) 20 angeordnet, der in einem mit dem Handgriff 2 zusammengebautem Zustand am distalen Schaftende des Schafts 10 des Handgriffs 2 aus demselben hervorragt. Der Crimpkopf 20 (nachfolgend auch als Maulteil bezeichnet) ist dazu eingerichtet, mit der als U-förmigen Schieber ausgebildeten Schließvorrichtung 12 des Handgriffs 2 zusammenzuwirken, um am distalen Ende des Clip-Applikators 1 gemeinsam das Crimpwerkzeug / Crimpmechanismus 22 des Clip-Applikators 1 auszubilden.

Für das Zusammenwirken mit der als U-förmigen Schieber ausgebildeten Schließvorrichtung 12 weist das Maulteil 20 (in seiner Längsrichtung gesehen) auf der linken und rechten Außenseite jeweils eine (Steuer-) Kulisse 24 und 26 auf. Die Kulissen 24, 26 und die Schließvorrichtung 12 sind jeweils dazu eingerichtet, gemeinsam eine Öffnungs- und Schließbewegung des Maulteils 20 zu definieren. Dabei sind die Position und die geometrische Form der Kulissen 24, 26 des Maulteils 20 für die unterschiedlichen Clip-Magazine aus der Vielzahl von Clip-Magazinen individuell festlegbar/festgelegt, insbesondere in Abhängigkeit des gewählten Clip-Magazins und/oder des zu applizierenden Cliptyps, da je nach Clip-Magazin und/oder Cliptyp eine größere/kleinere Maulöffnung am Crimpkopf 20 notwendig ist.

Die Öffnungs- und Schließbewegung des Crimpkopfs/Maulteils 20 ergibt sich, wenn die als U-förmiger Schieber ausgebildete Schließvorrichtung 12 bei deren Längsbewegung im Handgriff-Schaft 10 die Kulissen 24, 26 des Maulteils 20 wenigstens teilweise außenseitig umgreift, so dass zwei (in Figur 2 nicht gezeigte) Innenflächen der U-förmigen Schließvorrichtung 12 in Kontakt mit den Kulissen 24, 26 gelangen und damit auch die Öffnungs- und Schließbewegung des Maulteils 20 festlegen beziehungsweise steuern. Hierzu sind das Maulteil 20 und die Schließvorrichtung 12 jeweils dazu eingerichtet beziehungsweise konfiguriert, dass die als U-förmiger Schieber ausgebildete Schließvorrichtung 12 das mittels des Halteteils (Bolzens) 14 in Längsrichtung des Schafts 10 fixierte Maulteil 20 wenigstens teilweise umgreift, wenn die beiden Griffhebel 6 und 8 manuell betätigt (zusammen gedrückt) werden. Das heißt, dass die Schließvorrichtung 12 bei einer Betätigung der Griffhebel 6, 8 durch die vorstehend angedeuteten Pleuelstäbe 56, 58 des Handgriffs 2 längs in distaler Richtung des Clip-Applikators 1 so weit nach vorne in Richtung distal bewegt beziehungsweise geschoben wird, bis die Schließvorrichtung 12 die Kulissen 24, 26 des Maulteils 20 umgreift und dadurch das Maulteil 20 in eine Schließbewegung zwingt. Durch diese Bewegung der Schließvorrichtung 12, die durch die Position und/oder Form der Kulissen 24, 26 festgelegt beziehungsweise gesteuert wird, wird das Maulteil 20 geschlossen und nach erfolgter Betätigung der Griffhebel 6, 8 beziehungsweise durch ein Loslassen derselben wieder geöffnet. Die Öffnungsbewegung ergibt sich dabei durch eine (aus distaler Sicht) Rückwärtsbewegung der als U-förmigen Schieber ausgebildeten Schließvorrichtung 12.

Figur 3 zeigt eine perspektivische Unteransicht des Clip-Magazins 4, das zu Illustrationszwecken teilweise demontiert ist, beziehungsweise von dem einzelne Bauteile zur anschaulicheren Beschreibung entfernt sind.

In Figur 3 ist zu erkennen, dass das Crimpwerkzeug 22 im Wesentlichen gabelförmig ausgebildet ist und zwei zueinander parallele Branchen 28 und 30 aufweist. An den in distale Richtung weisenden freien Enden des Crimpwerkzeugs 22 weist dasselbe zwei einstückig angeformte Maulteilbacken 32 und 34 auf, an denen die oben beschriebenen Kulissen 24, 26 angeordnet/ausgebildet sind und welche das Maulteil (Crimpkopf) 20 bilden. Es ist ebenfalls zu erkennen, dass das Crimpwerkzeug 22 an seinem proximalen Ende eine Ausnehmung 36 als Befestigungsöffnung zum ineinandergreifenden Zusammenwirken mit dem Halteteil (Bolzen) 14 des Handgriffs 2 aufweist. Die Ausnehmung 36 ist in diesem Ausführungsbeispiel als Langloch ausgebildet, in das im zusammengesetzten Zustand das Halteteil 14 des Handgriffs 2 eingreift, um das in den Schaft 10 eingeclipste Crimpwerkzeug 22 derart sicher an dem Schaft 10 zu fixieren, dass es sich nicht in Längsrichtung des Clip-Magazins 4 beziehungsweise in Längsrichtung des Schafts 10 des Handgriffteils 2 bewegen kann. In anderen Worten ist die Längsposition des Crimpwerkzeugs 22 und damit des Maulteils 20 durch das Zusammenwirken der Ausnehmung 36 mit dem Halteteil 14 festgelegt, so dass es sich weder relativ zum Schaft 10 des Handgriffs 2, noch relativ zu anderen Bauteilen des Clip-Magazins 4 bewegen kann.

Das in Figur 3 dargestellte Clip-Magazin 4 weist auch eine Clip-Vorschubeinrichtung 38 in Form einer Clip-Vorschubstange zum Aufnehmen und Zustellen, das bedeutet Transportieren, von vereinzelten Clips auf. Die Clip-Vorschubeinrichtung 38 ist für ein Zusammenwirken mit dem Maulteil 20 eingerichtet und ist relativ zu diesem in Längsrichtung des Clip-Magazins 4 beziehungsweise in Längsrichtung des Schafts 10 des Handgriffs 2 verschiebbar gelagert. Das heißt, dass die Vorschubeinrichtung 38 die Funktion aufweist, einzelne Clips aus dem Clip-Magazin 4 aufzunehmen und diese in distaler Längsrichtung des Schafts 10 weiter zu transportieren. Hierfür weist die Clip-Vorschubeinrichtung 38 an seinem distalen Ende eine Clipzunge 40 auf, mit der einzelne Clips aufnehmbar und zustellbar beziehungsweise transportierbar sind.

Für die Aufnahme der einzelnen Clips aus dem Clip-Magazin 4 ist eine (aus distaler Sicht) Rückwärtsbewegung der Clip-Vorschubeinrichtung 38 notwendig, da die einzelnen Clips (aus distaler Sicht) hinter den Maulteilbacken 32, 34 zur Bevorratung positioniert sind. Aus diesem Grund muss die Clip-Vorschubeinrichtung 38 eine Rückwärtsbewegung ausführen, damit diese (aus distaler Sicht) hinter genau dem Clip positioniert wird, der als nächstes aufgenommen und in Längsrichtung des Schafts 10 nach vorne in Richtung hin zum Maulteil 20 zugestellt beziehungsweise transportiert werden soll. Dagegen ist für die Zustellung beziehungsweise den Transport des mittels der Clip-Vorschubeinrichtung 38 aufgenommen Clips eine (aus proximaler Sicht) Vorwärtsbewegung notwendig, um den aufgenommen Clip in Richtung hin zum Maulteil 20, das heißt entlang der Branchen 28, 30 des Crimpwerkzeugs 22 zwischen die Maulteilbacken 32, 34 desselben zu bewegen.

Um diese beiden Längsbewegungen der Clip-Vorschubeinrichtung 38, nämlich (aus proximaler Sicht) nach vorne hin zum Maulteil 20 und (aus proximaler Sicht) nach hinten hin zum Clipaufnahmeabschnitt (Clipspeicher) auf einfache Weise zu begrenzen, weist die Clip-Vorschubeinrichtung 38 zwei in Längsrichtung derselben voneinander beabstandete Begrenzungsanschläge 42 und 44 auf. Die Begrenzungsanschläge 42, 44 sind so ausgeformt und bemessen, dass sie jeweils mit dem Crimpwerkzeug 22 in

Kontakt kommen können, dessen Längsposition gegenüber der Clip-Vorschubeinrichtung 38 mittels des in die Ausnehmung 36 eingreifenden Halteteils 14 festgelegt ist. Das heißt, dass die am Crimpwerkzeug 22 situationsabhängig anschlagenden Begrenzungsanschläge 42, 44 die Längsbewegung der Clip-Vorschubeinrichtung 38 in beide Längsrichtungen begrenzen.

In diesem Ausführungsbeispiel sind die Begrenzungsanschläge 42, 44 als Stanz-/Biegeteil einstückig mit der Clip-Vorschubeinrichtung 38 ausgebildet. Der Abstand in Längsrichtung zwischen den beiden Begrenzungsanschlägen 42, 44 ist so bemessen beziehungsweise gewählt und festgelegt, dass die Clipzunge 40 bei einer (aus proximaler Sicht) Rückwärtsbewegung der Clip-Vorschubeinrichtung 38, das heißt bei einer Clip-Aufnahmebewegung der Clip-Vorschubreinrichtung 38, genau ein einziger Clip aus dem Clip-Magazin 4 aufgenommen wird, wie dies nachfolgend noch näher erläutert wird. In Figur 3 ist die Clip-Vorschubeinrichtung 38 in einer Lage/Position dargestellt, in der der proximale, das heißt der in Clip-Vorschubrichtung hintere Begrenzungsanschlag 44 mit dem Crimpwerkzeug 22 in anliegenden Kontakt ist. Gemäß Figur 3 ist die Clip-Vorschubeinrichtung 38 folglich in einer Lage/Position dargestellt, in der ein einzelner Clip 46 mittels der Clipzunge 40 der Clip-Vorschubeinrichtung 38 hin zum distalen Ende des Maulteils 20, das heißt in das Crimpzeug 22 hinein transportiert wurde beziehungsweise in diesem angeordnet ist.

Es ist offensichtlich, dass die in Figur 3 dargestellte Position der Clip-Vorschubeinrichtung 38 (noch) keine Position ist, in der sich die Backen 32, 34 des Maulteils 20 zum Vercrimpen des eingeführten Clip schließen können. Denn für das Schließen der Maulteilbacken 32, 34 ist vorab eine Rückwärtsbewegung der Clip-Vorschubeinrichtung 38 notwendig, damit diese nicht in der gezeigten Lage vollständig zwischen den Maulteilbacken 32, 34 angeordnet bleibt.

In Figur 4, die eine perspektivische Unteransicht eines Teilabschnitts des Clip-Magazins 4 in einem teilweise demontierten Zustand zeigt, ist die Clip-Vorschubeinrichtung 38 in einer anderen Lage/Position dargestellt, in der es den einzelnen Clip 46 aus einem Clipspeicherabschnitt 48 des Clip-Magazins 4 zum Zustellen beziehungsweise Weitertransport in Richtung hin zum Maulteil 20 (Crimpwerkzeug 22) aufnehmen kann. Es ist zu erkennen, dass der Clipspeicherabschnitt 48 (aus distaler Sicht) hinter den Maulteilbacken 32, 34 angeordnet ist, so dass ein einzelner Clip 46 (aus distaler Sicht) nach vorne zugestellt beziehungsweise transportiert werden muss, um ihn zwischen die Maulteilbacken 32, 34 zu fördern.

Weiter ist in Figur 4 zu erkennen, dass die Clip-Vorschubeinrichtung 38 in der dargestellten Clip-Aufnahmeposition mit dem distalen, das heißt mit dem in Clip-Vorschubrichtung vorderen Begrenzungsanschlag 42 an dem Crimpwerkzeug 22 anliegt, wodurch die Clip-Aufnahmebewegung in dieser Bewegungsrichtung begrenzt wird. Durch die in Abhängigkeit des Clip-Magazins 4 bemessene und entsprechend festgelegte Positionierung des Begrenzungsanschlags 42 ist sichergestellt, dass gleichzeitig immer nur ein einziger Clip 46 aus dem Clipspeicher 48 des Clip-Magazins 4 aufgenommen wird und dieser in einer anschließenden Clip-Zustellbewegung in Clip-Vorschubrichtung hin zum distalen Ende des Crimpwerkzeugs 22, das heißt zwischen die Maulteilbacken 32, 34 beziehungsweise in das Maulteil 20 hinein transportiert wird.

In Figur 5, die eine perspektivische Seitenansicht des Clip-Applikators 1 in einem teilweise demontierten Zustand zeigt, ist zu erkennen, dass der Handgriff 2 des Clip-Applikators 1 den integrierten Überlastungsschutz 50 mit Rutschkupplung 52 aufweist. Der Überlastschutz 50 mit Rutschkupplung 52) verhindert bei einem Anschlag des vorderen Begrenzungsanschlags 42 an dem Crimpwerkzeug 22, dass die Mechanik des Handgriffs 2 und/oder die Mechanik beziehungsweise Gehäuseteile des Clip-Magazins 4, oder auch der Begrenzungsanschlag 42 selbst beziehungsweise das Maulteil 20, durch zu hohe Betätigungskräfte des Anwenders beschädigt werden. Da die Abstände zwischen den Begrenzungsanschlägen 42, 44 des Clip-Magazins 4 für die unterschiedlichen Clip-Magazine aus der Vielzahl von Clip-Magazinen unterschiedlich positioniert beziehungsweise festgelegt sind, kann durch den Überlastungsschutz 50 zusätzlich sichergestellt werden, dass ein universaler Handgriff 2 mit mehreren aus der Vielzahl von Clip-Magazinen zusammenwirken kann, ohne die Mechanik oder die

Bauteile des jeweiligen Handgriffs 2 und/oder des jeweiligen Clip-Magazins 4 infolge zu hoher manueller Betätigungskräfte zu beschädigen.

Der Betrieb beziehungsweise der praktische Gebrauch des Clip-Applikators 1 kann wie nachfolgend beschrieben ablaufen.

Figur 2 zeigt in einer perspektivischen Seitenansicht den Clip-Applikator 1 im zusammengebauten, das heißt im funktionstüchtigen Zustand, in dem das Clip-Magazin 4 durch das Zusammenwirken der Montagevorrichtung 16 und der Haltevorrichtung 18 aneinander befestigt sind.

Nach der Montage des Clip-Applikators 1 durch das Befestigen des bedarfsweise ausgewählten Clip-Magazins 4 an dem universalen Handgriff 2, befindet sich der Clip-Applikator 1 in einem funktionstüchtigen Zustand. D.h. in diesem Zustand ist das Clip-Magazin 4 in den Handgriff-Schaft 10 eingesetzt und wird vom Riegel 18 arretiert.

Durch eine manuelle Betätigung der zwei Griffhebel 6 und 8 des Handgriffs 2 wird die interne Betätigungsmechanik 54 des Handgriffs 2 so aktiviert, dass die als U-förmiger Schieber ausgebildete Schließvorrichtung 12 in distale Richtung längsverschoben wird. Bedingt durch diese Längsverschiebung der Schließvorrichtung 12 wird diese über die Kulissen 24, 26 des Maulteils 20 geschoben, wodurch dessen Maulteilbacken 32, 34 aufeinander zu, d.h. nach innen gedrückt werden und somit das Crimpwerkzeug 22 geschlossen wird.

Andererseits (gleichzeitig) wird die interne Betätigungsmechanik 54 des Handgriffs 2 auch so betätigt, dass die Clip-Vorschubeinrichtung 38 in einer der Schließvorrichtung 12 gegenläufigen Längsbewegung (in Richtung proximal) solange verschoben wird, bis der vordere Begrenzungsanschlag 42 am fixierten Crimpwerkzeug 22 anschlägt und gegebenenfalls die Rutschkupplung 52 des Überlastungsschutzes 50 ausgelöst wird. Die interne Betätigungsmechanik 54 des Handgriffs 2 hält die Clip-Vorschubeinrichtung 38 dann solange in dieser Position fest, bis die Maulteilbacken 32, 34 durch ein Zurückverschieben der Schließvorrichtung 12 wieder voneinander beabstandet warden und somit das Crimpwerkzeug 22 wieder geöffnet wird. Somit ist nun wieder ein Freiraum zwischen den Maulteilbacken 32, 34 geschaffen, wobei die Clip-Vorschubeinrichtung 38 mittels der internen Betätigungsmechanik 54 (automatisch) freigegeben wird und durch eine feder-vorgespannt unterstützte Vorwärtsbewegung der nächste Clip durch die Clipzunge 40 zwischen Maulteilbacken 32, 34 transportiert werden kann.

Zusammenfassend wird ein chirurgischer Clip-Applikator offenbart, mit einem Universal-Handgriff und mit einem Individual-Clipmagazin mit integrietem Crimpkopf/Crimpwerkzeug, das an dem Universal-Handgriff lösbar befestigbar ist. Der Universal-Handgriff und das daran befestigte Individual-Clipmagazin wirken zusammen, um einen zur Betätigung festgelegten bzw. individuell zur Verfügung stehenden Zustellweg für den Cliptransport einzelner Clips aus einem Clip-Aufnahmeabschnitt des Clip-Magazins hin zum Crimpkopf auszubilden. Erfindungsgemäß ist das Individual-Clipmagazin aus einer Vielzahl von Clip-Magazinen für unterschiedliche Cliptypen bedarfsweise auswählbar, wobei das Clip-Magazin eine integrierte und in Abhängigkeit des zu applizierenden Cliptyps ausgebildete Kodiermechanik aufweist, die im Zusammenwirken mit den Universal-Handgriff wenigstens den an den jeweiligen Cliptyp angepassten Zustellweg festlegt.

## Patentansprüche

1. Universalhandgriff eines chirurgischen Clip-Applikators (1), an dem ein einen Crimpkopf/Crimpwerkzeug (20, 22) aufweisendes, aus einer Anzahl von unterschiedlichen Clipmagazinen beliebig auswählbares Individual-Clipmagazin (4) montier- oder befestigbar ist, derart, dass der Universalhandgriff (2) und das daran befestigte Individual-Clipmagazin (4) zum Vercrimpen einzelner Clips (46) im Crimpkopf/Crimpwerkzeug (20, 22) sowie zum Fördern einzelner Clips aus einem Clip-Aufnahmeabschnitt (48) über einen individuellen Zustellweg hin zum Crimpkopf/Crimpwerkzeug (20, 22) mechanisch zusammenwirken, wofür der Universalhandgriff (2) ein integriertes Kraftübertragungsgetriebe (54) mit wenigstens einer manuell ergreifbaren Handhabe (6, 8) als Betätigungs-Eingangsteil und mit einer Kopplung als Betätigungs-Abgabeteil zum Individual-Clipmagazin (4) hat,
**gekennzeichnet durch** eine Adaptionsvorrichtung (50, 52) mit einem Überlastungsschutz (50), der zwischen Betätigungs-Eingangsteil und Betätigungs-Abgabeteil für ein Unterbrechen der Kraftübertragung im Kraftübertragungsgetriebe (54) angeordnet ist, wenn die Förderung einzelner Clips aus dem Clipmagazin (4) durch zumindest einen Begrenzungsanschlag (42, 44) begrenzt ist, zur Kompensation/Anpassung von Clip-bedingten Unterschieden zwischen dem individuellen Zustellweg des aktuell befestigten Individual-Clipmagazins (4) und einem maximalen Betätigungsweg des Betätigungs-Eingangsteils am Universalhandgriff (2).

2. Universalhandgriff nach Anspruch 1, **dadurch gekennzeichnet, dass** die Adaptionsvorrichtung (50, 52) dafür ausgebildet ist, dass der an den jeweiligen Cliptyp angepasste Zustellweg durch eine in dem aktuell befestigten Individual-Clipmagazin (4) integrierte sowie in Abhängigkeit des zu applizierenden Cliptyps ausgebildete Kodiermechanik (20, 24, 26, 38, 42, 44) festgelegt wird oder ist.

3. Universalhandgriff nach Anspruch 1 oder 2, **dadurch gekennzeichnet, dass** der Überlastungsschutz (50) eine Rutschkupplung (52) oder einen Ausklinkmechanismus aufweist.

4. Universalhandgriff nach Anspruch 3, **dadurch gekennzeichnet, dass** die Rutschkupplung (52) auf einen Durchrutschwert oberhalb der für die Förderung einzelner Clips aus dem Clipaufnahmespeicher (48) über den individuellen Zustellweg hin zum Crimpkopf/Crimpwerkzeug (20, 22) erforderlichen Kraft sowie unterhalb einer vorbestimmten, das Kraftübertragungsgetriebe (54) überbelastenden Betätigungskraft eingestellt ist.

5. Chirurgischer Clip-Applikator (1), **gekennzeichnet durch**
einen Universal-Handgriff (2) gemäß einem der Ansprüche 1 bis 4 und
ein separat dazu und zur Montage an dem Universalhandgriff (2) ausgebildeten Individual-Clipmagazin (4), welches
einen Crimpkopf /Crimpwerkzeug (20, 22),
einen Clip-Aufnahmeabschnitt (48) und
eine Clip-Vorschubeinrichtung (38) zum Fördern einzelner Clips aus dem Clip-Aufnahmeabschnitt (48) über einen individuellen Zustellweg hin zum Crimpkopf/Crimpwerkzeug (20, 22) in Abhängigkeit einer manuellen Betätigung des Universalhandgriffs (2) aufweist, wobei die Clip-Vorschubeinrichtung (38) dafür angepasst ist, um mit einem Rast-/Eingreifabschnitt am distalen Ende des Betätigungs-Abgabeteils/Ausgangselements an der Adaptionsvorrichtung (52) des Universalhandgriffs zusammen zu wirken, und wobei
das aus einer Vielzahl von Clipmagazinen für unterschiedliche Cliptypen bedarfsweise auswählbare Individual-Clipmagazin (4) eine integrierte und in Abhängigkeit des zu applizierenden Cliptyps ausgebildete Kodiermechanik (20, 24, 26, 38, 42, 44) mit zwei Begrenzungsanschlägen (42, 44) zur wenigstens teilweisen Festlegung des an den jeweiligen Cliptyp angepassten, individuellen Zustellwegs aufweist, um die Bewegung der Clip-Vorschubeinrichtung (38) in ihre beiden Längsrichtungen zu begrenzen.

6. Chirurgischer Clip-Applikator (1) nach Anspruch 5, **dadurch gekennzeichnet, dass** die Kodiermechanik zwei in Längs-/Bewegungsrichtung der Clip-Vorschubeinrichtung voneinander beabstandete Begrenzungsanschläge (42, 44) aufweist, deren Positionen in Abhängigkeit des zu applizierenden Cliptyps derart angepasst sind, dass durch eine Rückwärts- und/oder Vorwärtsbewegung der Vorschubeinrichtung (38) jeweils genau ein Clip aus dem Clip-Magazin (4) hin zum Crimpkopf/Crimpwerkzeug (20, 22) transportierbar ist.

7. Chirurgischer Clip-Applikator (1) nach Anspruch 5 oder 6, **dadurch gekennzeichnet, dass** die Clip-Vorschubeinrichtung (38) ein längsbeweglich gehaltener Stab vorzugsweise in Form einer Platte ist, wobei weiter vorzugsweise der wenigstens eine Begrenzungsanschlag (42, 44) als ein Schnitt- und/oder Biegeteil in den Stab integriert ist.

8. Chirurgischer Clip-Applikator (1) nach Anspruch 5 oder 6, **dadurch gekennzeichnet, dass** der Crimpkopf/Crimpwerkzeug (20, 22) angepasst ist, um mit dem wenigstens einen Begrenzungsanschlag (42, 44) der Clip-Vorschubeinrichtung (38) in Kontakt zu kommen, um eine Rückwärts- und/oder Vorwärtsbewegung der Clip-Vorschubeinrichtung (38) zu begrenzen.

9. Chirurgischer Clip-Applikator (1) nach einem der Ansprüche 5 bis 8, **dadurch gekennzeichnet, dass** der Universal-Handgriff (2) als ein wiederverwendbares Teil ausgebildet ist.

10. Chirurgischer Clip-Applikator (1) nach einem der Ansprüche 5 bis 9, **dadurch gekennzeichnet, dass** das Individual-Clipmagazin (4) als ein Einweg-Teil ausgebildet ist.

11. Chirurgischer Clip-Applikator (1) nach einem der vorangehenden Ansprüche 5 bis 10, **dadurch gekennzeichnet, dass** der Universal-Handgriff (2) und das Individual-Clipmagazin (4) eine an den zu applizierenden Cliptyp angepasste Farb- und/oder Symbolkodierung (5a, 5b) zur Visualisierung des mit dem Universal-Handgriff (2) aktuell verwendeten Individual-Clipmagazins (4) aufweist.

12. Chirurgischer Clip-Applikator (1) nach einem der vorangehenden Ansprüche 5 bis 11, **dadurch gekennzeichnet, dass** der Crimpkopf /Crimpwerkzeug (20, 22) eine das Schließen und Öffnen steuernde Kulisse aufweist, deren Position und/oder Geometrie an den zu applizierenden Cliptyp und/oder an die Schaftlänge des Clip-Magazins (4) angepasst ist.

## Claims

1. A universal handgrip of a surgical clip applicator (1) to which an individual clip magazine (4), which comprises a crimping head/crimping tool (20, 22) and can be arbitrarily selected from a number of different clip magazines, can be mounted or fastened such that the universal handgrip (2) and the individual clip magazine (4) fastened thereto mechanically interact for crimping individual clips (46) in the crimping head/crimping tool (20, 22) as well as for conveying individual clips from a clip receiving portion (48) via an individual feed path toward the crimping head/crimping tool (20, 22), for which purpose the universal handgrip (2) is provided with an integrated force transmission gearing (54) comprising at least one manually graspable handle (6, 8) as an actuation-/input part and comprising a coupling means as an actuation-/delivery part to the individual clip magazine (4),
**characterized by** an adaptation device (50, 52) with an overload protection means arranged between the actuation-/input part and the actuation-/delivery part for interruption of the power transmission in the force transmission gearing (54) when delivery of individual clips from the clip magazine (4) is limited by at least one limiting stops (42, 44), for compensating and accommodating clip-related differences between the individual feed path of the currently mounted individual clip magazine (4) and a maximum actuating travel of the actuation-/input part on the universal handgrip (2).

2. The universal handgrip according to claim 1, **characterized in that** the adaptation device (50, 52) is designed for the purpose that the feed path adapted to the respective clip type is defined by a coding mechanism (20, 24, 26, 38, 42, 44) which is integrated in the currently mounted individual clip magazine (4) and formed depending on the clip type to be applied.

3. The universal handgrip according to claim 1 or 2, **characterized in that** the overload protection means (50) comprises a sliding clutch (52) or a releasing mechanism.

4. The universal handgrip according to claim 3, **characterized in that** the sliding clutch (52) is adjusted so as to have a slipping value which is above the force required for conveying individual clips from the clip receiving reservoir (48) via the individual feed path toward the crimping head/crimping tool (20, 22) as well as below a predetermined actuating force overloading the force transmission gearing (54).

5. A surgical clip applicator (1), **characterized by**
a universal handgrip (2) according to one of claims 1 to 4, and
an individual clip magazine formed separately and to be mounted to the universal handgrip (2), comprising
a crimping head/crimping tool (20, 22),
a clip receiving portion (48), and
a clip advancing means (38) for conveying individual clips from the clip receiving portion (48) via an individual feed path toward the crimping head/crimping tool (20, 22) depending on a manual operation of the universal handgrip (2), wherein the clip advancing means (38) is adapted to cooperate with a latching/engaging portion at the distal end of the actuation input part/delivery part on the adaptation device (52) of the universal handle, and wherein
the individual clip magazine (4), which can be selected from a number of clip magazines for different clip types as needed, comprises an integrated coding mechanism (20, 24, 26, 38, 42, 44), which is formed depending on the clip type to be applied and has two limiting stopss (42, 44) for at least partially determining the individual feed path adapted to the respective clip type, in order to limit the movement of the clip advancing means (38) in its two longitudinal directions

6. The surgical clip applicator (1) according to claim 5, **characterized in that** the coding mechanism comprises two limiting stops (42, 44) which are spaced from each other in the longitudinal direction or the direction of movement of the clip advancing means, the positions of said limiting stops being adapted depending on the clip type to be applied such that a rearward and/or forward movement of the clip advancing means (38) allows to transport exactly one clip from the clip magazine (4) toward the crimping head/crimping tool (20, 22).

7. The surgical clip applicator (1) according to claim 5 or 6, **characterized in that** the clip advancing means (38) is a rod which is supported to be movable in the longitudinal direction and is realized preferably in the form of a plate, further preferably the at least one limiting stop (42, 44) being integrated in the rod as a cut and/or bent part.

8. The surgical clip applicator (1) according to claim 5 or 6, **characterized in that** the crimping head/crimping tool (20, 22) is adapted to come into contact with the at least one limiting stop (42, 44) of the clip advancing means (38) in order to limit a rearward and/or forward movement of the clip advancing means (38).

9. The surgical clip applicator (1) according to one of claims 5 to 8, **characterized in that** the universal handgrip (2) is designed as a reusable part.

10. The surgical clip applicator (1) according to one of claims 5 to 9, **characterized in that** the individual clip magazine (4) is designed as a disposable part.

11. The surgical clip applicator (1) according to one of the preceding claims 5 to 10, **characterized in that** the universal handgrip (2) and the individual clip magazine (4) comprises a color-based and/or symbol-based coding (5a, 5b) which is adapted to the clip type to be applied and intended for visualizing the individual clip magazine (4) currently used with the universal handgrip (2).

12. A surgical clip applicator (1) according to one of the preceding claims 5 to 11, **characterized in that** the crimping head/crimping tool (20, 22) has a gate controlling closing and opening, the position and/or geometry of which is adapted to the type of clip to be applied and/or to the shaft length of the clip magazine (4).

## Revendications

1. Poignée universelle d'un applicateur de clips chirurgicales (1) à laquelle un magasin de clips individuels (4) comprenant une tête de sertissage/un outil de sertissage (20, 22) et pouvant être choisi à volonté parmi un certain nombre de magasins de clips différents, peut être monté ou fixé de telle sorte que la poignée universelle (2) et le magasin de clips individuels (4) fixé à celle-ci coopèrent mécaniquement pour sertir des clips individuels (46) dans la tête de sertissage/l'outil de sertissage (20, 22) et pour transporter des clips individuels depuis une section de réception de clips (48) sur un trajet d'avancement individuel vers la tête de sertissage/l'outil de sertissage (20, 22), pour lequel la poignée universelle (2) a un mécanisme de transmission de puissance intégrée (54) avec au moins une poignée pouvant être saisie manuellement (6, 8) comme partie d'entrée d'actionnement et avec un accouplement comme partie de sortie d'actionnement au magasin de clips individuels (4), **caractérisé par** un dispositif d'adaptation (50, 52) avec une protection contre les surcharges (50) disposé entre la partie d'entrée d'actionnement et la partie de sortie d'actionnement pour interrompre la transmission de puissance dans le mécanisme de transmission de puissance (54), lorsque la sortie des clips individuels du magasin de clips (4) est limitée par au moins une butée (42, 44), pour compenser/régler les différences liées aux clips entre le trajet de sortie individuelle du magasin de clips individuels (4) actuellement monté et une trajet d'actionnement maximale de la partie d'entrée d'actionnement sur la poignée universelle (2).

2. Poignée universelle selon la revendication 1, **caractérisée en ce que** le dispositif d'adaptation (50, 52) est conçu de telle sorte que le trajet d'avancement adapté au type de clip respectif est déterminé par un mécanisme de codage (20, 24, 26, 38, 42, 44) intégré dans le magasin de clips individuels (4) actuellement monté et conçu en fonction du type de clip à appliquer.

3. Poignée universelle selon la revendication 1 ou 2, **caractérisée en ce que** la protection contre les surcharges (50) comprend un embrayage à glissement (52) ou un mécanisme de libération.

4. Poignée universelle selon la revendication 3, **caractérisée en ce que** l'embrayage à glissement (52) est réglé à une valeur de glissement supérieure à la force nécessaire pour transporter des clips individuels depuis le magasin de réception de clips (48) sur le trajet d'avancement individuel vers la tête de sertissage/l'outil de sertissage (20, 22) et inférieure à une force d'actionnement prédéterminée surchargeant le mécanisme de transmission de puissance (54).

5. Applicateur de clips chirurgicales (1) **caractérisé par**
une poignée universelle (2) selon l'une quelconque des revendications 1 à 4 et
un magasin de clips individuels (4) séparé de celle-ci et adapté pour être monté sur la poignée universelle (2), le magasin de clips (4) comprenant
une tête de sertissage/un outil de sertissage (20, 22),
une section de réception de clips (48) et
un dispositif d'avancement de clips (38) pour transporter des clips individuels depuis la section de réception de clips (48) sur un trajet d'avancement individuel vers la tête de sertissage/outil de sertissage (20, 22) en réponse à un actionnement manuel de la poignée universelle (2), dans lequel le dispositif d'avancement de clips (38) est adapté pour coopérer avec une partie de verrouillage/d'engagement à l'extrémité distale de la partie de sortie de l'actionneur/élément de sortie sur le dispositif d'adaptation (52) de la poignée universelle, et dans
lequel le magasin de clips individuels (4), pouvant être sélectionné parmi une pluralité de magasins de clips pour différents types de clips selon les besoins, a un mécanisme de codage intégré (20, 24, 26, 38, 42, 44) formé en fonction du type de clip à appliquer, avec deux butées (42, 44) pour définir au moins partiellement la trajet d'avancement individuel adapté au type de clip respectif afin de limiter le mouvement du dispositif d'alimentation de clips (38) dans ses deux directions longitudinales.

6. Applicateur de clip chirurgical (1) selon la revendication 5, **caractérisé en ce que** le mécanisme de codage comprend deux butées de limitation (42, 44) qui sont espacées l'une de l'autre dans la direction longitudinale/déplacement du dispositif d'avancement des clips et dont les positions sont adaptées en fonction du type de clip à appliquer de telle sorte que, par un mouvement de recul et/ou d'avance, le dispositif d'alimentation de clips (38) peut être déplacé en arrière et/ou en avant et un seul clip à la fois peut être transporté du magasin de clips (4) vers la tête de sertissage/l'outil de sertissage (20, 22).

7. Applicateur de clip chirurgical (1) selon la revendication 5 ou 6, **caractérisé en ce que** le dispositif d'avancement des clips (38) est une tige maintenue mobile longitudinalement, de préférence sous la forme d'une plaque, dans lequel, en outre, de préférence, la au moins une butée (42, 44) est intégrée en tant qu'élément de coupe et/ou de flexion dans la tige.

8. Applicateur de clip chirurgical (1) selon la revendication 5 ou 6, **caractérisé en ce que** la tête de sertissage/l'outil de sertissage (20, 22) est adaptée pour entrer en contact avec la au moins une butée de limitation (42, 44) du dispositif d'avancement des clips (38) pour limiter le mouvement du dispositif d'avancement des clips (38) vers l'arrière et/ou vers l'avant

9. Applicateur de clip chirurgical (1) selon l'une quelconque des revendications 5 à 8, **caractérisé en ce que** la poignée universelle (2) est conçue comme une pièce réutilisable.

10. Applicateur de clip chirurgicals (1) selon l'une des revendications 5 à 9, **caractérisé en ce que** le magasin de clips individuels (4) est conçu comme une pièce jetable.

11. Applicateur de clip chirurgical (1) selon l'une des revendications précédentes 5 à 10, **caractérisé en ce que** la poignée universelle (2) et le magasin de clips individuels (4) présentent un code de couleurs et/ou de symboles (5a, 5b) adapté au type de clip à appliquer pour visualiser le magasin de clips individuels (4) actuellement utilisé avec la poignée universelle (2).

12. Applicateur de clip chirurgical (1) selon l'une des revendications précédentes 5 à 11, **caractérisé en ce que** la tête de sertissage/l'outil de sertissage (20, 22) comporte un mécanisme de commande de fermeture et d'ouverture dont la position et/ou la géometrie est adaptée au type de clip à appliquer et/ou à la longueur de la tige du magasin de clips (4).
